# EUROPEAN PATENT SPECIFICATION

(11) **EP 2 403 508 B1**
(45) Date of publication and mention of the grant of the patent: **28.10.2015**
(21) Application number: 10706679.7
(22) Date of filing: 08.03.2010
(51) Int. Cl.: C12N 5/09, A61K 35/12

(54) **METHOD OF TREATING CANCER EMPLOYING GINGIVAL FIBROBLAST CONDITIONED MEDIUM**
VERFAHREN ZUR KREBSBEHANDLUNG MIT KONDITIONIERTEM KULTURMEDIUM AUS ZAHNFLEISCH-FIBROBLASTEN
METHODE POUR LE TRAITMENT DU CANCER PAR UN MILIEU DE CULTURE CONDITIONNÉS DERIVÉE DE FIBROBLASTES DE GENCIVES

(30) Priority: 06.03.2009 US 158037 P
(43) Date of publication of application: 11.01.2012
(73) Proprietor: Université Paris Descartes, 75270 Paris Cedex 06 (FR); Assistance Publique - Hôpitaux de Paris, 75184 Paris Cedex 04 (FR); Inserm, 75654 Paris Cedex 13 (FR); Ministère de la Défense Service de Santé des Armées, 92141 Clamart Cedex (FR)
(72) Inventor: GOGLY, Bruno, F-77510 Hondevilliers (FR); LAFONT, Antoine, F-75007 Paris (FR); COULOMB, Bernard, F-91430 Igny (FR); LATAILLADE, Jean-Jacques, F-78990 Elancourt (FR)
(74) Representative: Vial, Lionel
(86) International application number: PCT/EP2010/052901
(87) International publication number: WO 2010/100282

(56) References cited:
- WO-A2-2006/013261
- WO-A2-2008/017927
- DONG ZHONG ET AL: "Differential regulation of matrix metalloproteinase-9, tissue inhibitor of metalloproteinase-1 (TIMP-1) and TIMP-2 expression in co-cultures of prostate cancer and stromal cells" INTERNATIONAL JOURNAL OF CANCER, vol. 93, no. 4, 15 August 2001 (2001-08-15), pages 507-515, XP9132422 ISSN: 0020-7136
- BAR-YEHUDA S ET AL: "Resistance of muscle to tumor metastases: a role for a3 adenosine receptor agonists." NEOPLASIA (NEW YORK, N.Y.) 2001 MAR-APR LNKD- PUBMED:11420748, vol. 3, no. 2, March 2001 (2001-03), pages 125-131, XP09024521 ISSN: 1522-8002
- OHMORI Y ET AL: "Spontaneous production of thymocyte-activating factor by human gingival fibroblasts and its autoregulatory effect on their proliferation." INFECTION AND IMMUNITY APR 1987 LNKD- PUBMED:3549566, vol. 55, no. 4, April 1987 (1987-04), pages 947-954, XP9132406 ISSN: 0019-9567
- PARK HO ET AL: "Hyperthermia on mesenchymal stem cells (MSCs) can sensitize tumor cells to undergo cell death." INTERNATIONAL JOURNAL OF HYPERTHERMIA : THE OFFICIAL JOURNAL OF EUROPEAN SOCIETY FOR HYPERTHERMIC ONCOLOGY, NORTH AMERICAN HYPERTHERMIA GROUP DEC 2008 LNKD- PUBMED:19065344, vol. 24, no. 8, December 2008 (2008-12), pages 638-648, XP9132417 ISSN: 1464-5157
- KOONTONGKAEW SITTICHAI ET AL: "Tumor-stroma interactions influence cytokine expression and matrix metalloproteinase activities in paired primary and metastatic head and neck cancer cells" CELL BIOLOGY INTERNATIONAL, vol. 33, no. 2, February 2009 (2009-02), pages 165-173, XP25924779 ISSN: 1065-6995
- GOGLY BRUNO ET AL: "Preservation of aorta elastin network degradation by the presence of gingival fibroblasts: A cellular therapy strategy for the treatment of aneurysms" WOUND REPAIR AND REGENERATION, vol. 15, no. 6, November 2007 (2007-11), page A120, XP2578709 & 17TH ANNUAL MEETING OF THE EUROPEAN-TISSUE-REPAIR-SOCIETY; SOUTHAMPTON, UK; SEPTEMBER 26 -28, 2007 ISSN: 1067-1927
- HILL STEVEN J ET AL: "The effect of lipopolysaccharide on growth factor-induced mitogenesis in human gingival fibroblasts" JOURNAL OF PERIODONTOLOGY, AMERICAN ACADEMY OF PERIODONTOLOGY, CHICAGO, IL, US, vol. 67, no. 12, 1 January 1996 (1996-01-01), pages 1274-1280, XP009102159 ISSN: 0022-3492

## Description

### Field of the invention

The present invention relates to a method for treating cancer, in particular by inhibiting tumor invasion.

### Background of the invention

Tumor invasion is a key step in cancer progression, in which malignant cells with high invasive potential diffuse trough the basal lamina and form metastases. Accordingly, tumor invasion is one of the most important targets for designing treatments against metastastic cancers.

Among innovative strategies potentially useful for inhibiting cancer progression, therapy using cell-derived products, such as conditioned media, seems promising but has still not been soundly assessed.

Thus, Bar-Yehuda et al. (1999) Clin. Exp. Metastasis 17:531-535, following the observation that cancer rarely arose from skeletal muscle tissues, have shown that skeletal muscle cell conditioned medium administered to mice inoculated intravenously with melanoma or sarcoma cells, resulted in a statistically significant inhibition of metastatic lung foci. However, this treatment has not been further assessed in a clinical setting.

Besides, depending on the cell type, opposite results have been reported. In this regard, Chen et al. (2005) Surgery 138:382-90, in an attempt at determining how stromal microenvironment influences tumor progression, have shown that normal myofibroblasts or conditioned medium from normal myofibroblasts enhanced proliferation of colon cancer cells.

Gingival fibroblasts synthesise collagens (e.g. types I, III, V, VI, VII, XII), elastic fibers (oxytalan, elaunin and elastin), proteoglycans and glycosaminoglycans (e.g. decorin, biglycan), and glycoproteins (e.g. fibronectin, tenascin). Simultaneously, gingival fibroblasts synthesise enzymes that are able to degrade the macromolecular compounds (matrix metalloproteinases; MMPs), but also enzymes inhibiting active forms of MMPs (Inhibitors of metalloproteinases; TIMPs). Accordingly, gingival fibroblasts are important actors of extracellular matrix remodelling, either contributing to its synthesis or degradation.

### Summary of the invention

The present invention arises from the unexpected finding, by the inventors, that the conditioned medium of gingival fibroblasts inhibits malignant cell invasion *ex vivo.*

Thus, the present invention relates to a method for preventing or treating cancer in an individual, comprising administering the individual with a prophylactically or therapeutically effective quantity of a gingival fibroblast-derived product.

The present invention also relates to a gingival fibroblast-derived product for use in the prevention or treatment of cancer in an individual.

### Description of the figures

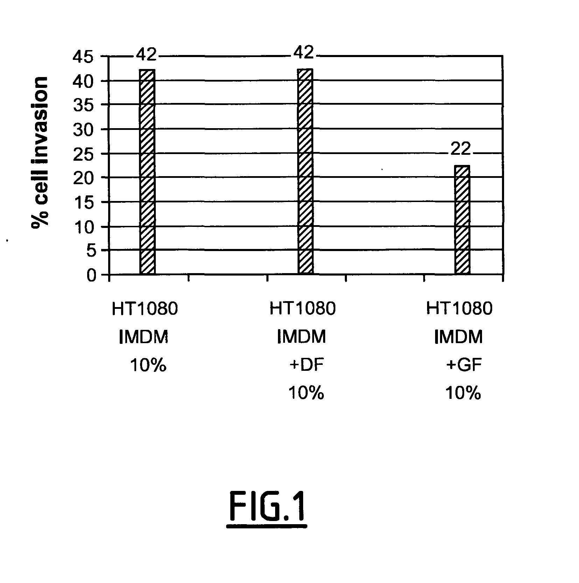

Figure 1 represents cell invasion of basal lamina extracts (vertical axis, in percentage) by HT1080 cells cultivated in IMDM medium with 10% FCS (HT1080 IMDM 10%), IMDM medium added with conditioned medium of human dermal fibroblasts cultivated with 10% FCS (HT1080 IMDM+ DF 10%), IMDM medium added with conditioned medium of human gingival fibroblasts cultivated with 10% FCS (HT1080 IMDM+ GF 10%).

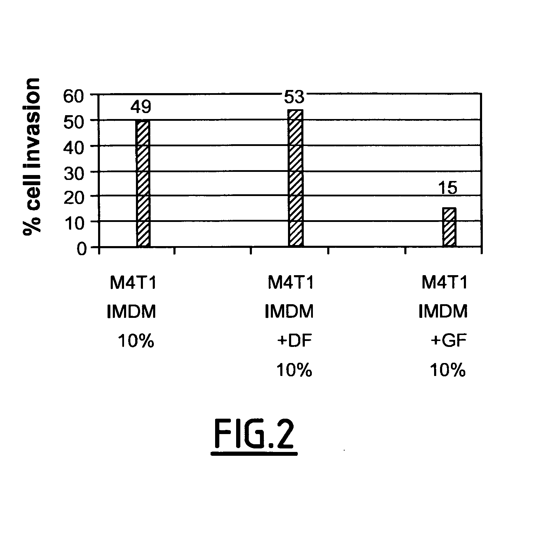

Figure 2 represents cell invasion of basal lamina extracts (vertical axis, in percentage) by M4T1 cells cultivated in IMDM medium with 10% FCS (M4T1 IMDM 10%), IMDM medium added with conditioned medium of human dermal fibroblasts cultivated with 10% FCS (M4T1 IMDM+ DF 10%), IMDM medium added with conditioned medium of human gingival fibroblasts cultivated with 10% FCS (M4T1 IMDM+ GF 10%).

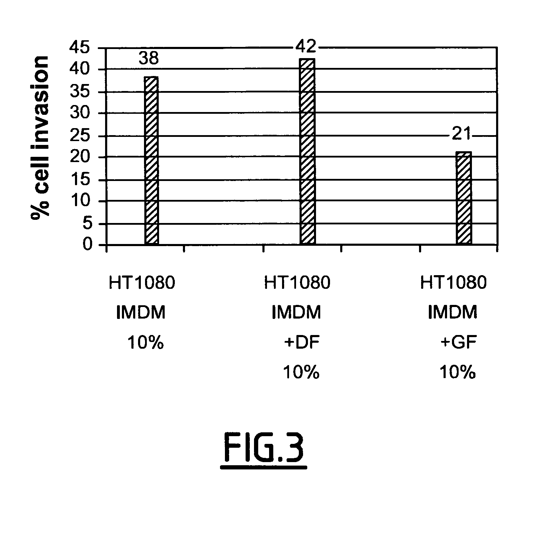

Figure 3 represents cell invasion of collagen I (vertical axis, in percentage) by HT1080 cells cultivated in IMDM medium with 10% FCS (HT1080 IMDM 10%), IMDM medium added with conditioned medium of human dermal fibroblasts cultivated with 10% FCS (HT1080 IMDM+ DF 10%), IMDM medium added with conditioned medium of human gingival fibroblasts cultivated with 10% FCS (HT1080 IMDM+ GF 10%).

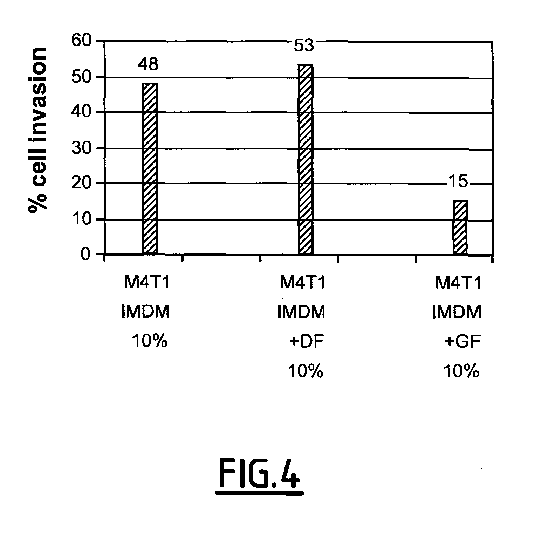

Figure 4 represents cell invasion of collagen I (vertical axis, in percentage) by M4T1 cells cultivated in IMDM medium with 10% FCS (M4T1 IMDM 10%), IMDM medium added with conditioned medium of human dermal fibroblasts cultivated with 10% FCS (M4T1 IMDM+ DF 10%), IMDM medium added with conditioned medium of human gingival fibroblasts cultivated with 10% FCS (M4T1 IMDM+ GF 10%).

### Detailed description of the invention

As intended herein the cancer to be prevented or treated according to the invention can be of any type. Preferably, it is a metastatic cancer or a cancer liable to form metastasis. Thus, preferably in the method of the invention, cancer cell invasion is inhibited. Furthermore, where the cancer forms tumors, in particular solid tumors, the method of the invention preferably prevents or treats tumor invasion. In other words, the method of the invention preferably prevents or treats metastasis of the cancer.

As intended herein the cancer preferably is a cancer of connective tissues. More preferably, the cancer is selected from the group consisting of breast cancer, and fibrosarcoma.

Preferably the individual is a mammal and more preferably a human.

Procedures for taking, culturing and preserving gingival fibroblasts are well known to the man skilled in the art and are particularly described in Naveau et al. (2006) J. Periodontol. 77:238-47 and in Gogly et al. (2007) Arterioscler. Thromb. Vasc. Biol. 27:1984-90.

Advantageously, gingival fibroblasts are easily sampled and cultured. Besides, gingival fibroblasts possess a high expansion rate.

Preferably, the gingival fibroblasts used in the method according to the invention are autologous, that is they are taken from the individual, to whom the gingival fibroblast-derived product is intended to be administered.

Advantageously, gingival fibroblasts provide for an almost limitless source of autologous fibroblasts. Furthermore, in case of aged skin, culture-competent autologous gingival fibroblasts are usually still available, whereas, in contrast, sources of culture-competent autologous dermal fibroblasts are scarce.

However, the gingival fibroblasts can also be allogenic, that is taken from another individual of the same species or heterologous, that is taken from another individual of another species.

As intended herein "gingival fibroblast-derived product" relates to any product which can be obtained from gingival fibroblasts in themselves or which contains gingival fibroblasts secretions. For example, it is preferred that the gingival fibroblast derived product is selected from the group consisting of gingival fibroblast whole cells, a gingival fibroblast culture, a gingival fibroblast extract, and a gingival fibroblast conditioned medium.

Gingival fibroblast extracts can be obtained by any cell fragmentation method known in the art.

Gingival fibroblast conditioned medium relates to any medium, such as a liquid cell culture medium, which has been contacted by gingival fibroblasts, in particular for a time sufficient for the gingival fibroblasts to have secreted in the medium.

Administration of the gingival fibroblast-derived product can proceed by any method known in the art. Thus, the gingival fibroblast-derived product can be injected locally, i.e. at a site near the tumor to be treated, or directly into the tumor to be treated. Besides, the gingival fibroblast-derived product can be administered by a route selected from the group consisting of the oral route, the subcutaneous route, the intravenous route, and the intramuscular route.

Preferably, the method according to the invention comprises the following steps:
- taking gingival fibroblasts from the individual;
- culturing the gingival fibroblasts;
- obtaining a gingival fibroblast-derived product from the cultured gingival fibroblasts;
- administering the gingival fibroblast-derived product to the individual.

### EXAMPLES

### Methods

### 1. Cell culture

Malignant cells were obtained from the 4T1 cell line of murine breast carcinoma (M4T1) or from the HT1080 cell line of human fibrosarcome.

Conditioned medium is obtained after 24 hours of culture of 2 millions of gingival or dermal fibroblasts in 5 ml of Iscove's Modified Dulbecco's Medium (IMDM) (GIBCO ref:12440) with 10% of foetal calf serum (FCS) (GIBCO ref:1600-044) at 37°C in **5%** CO₂.

### 2. Cellular invasion test

60000 4T1 or HT1080 cells are cultivated with 10% FCS in a cellular invasion kit of basal lamina extracts (R&D system ref: 3455-96-K) or of type I collagen (R&D system ref: 3457-96-K) (50µl) and optionally brought into contact with conditioned media obtained as described above (150µl). Cellular invasion is measured after 24 hours according to the manufacturer instructions.

### Results

### 1. Conditioned medium from human gingival fibroblasts inhibit cellular invasion by malignant cells on basal lamina extracts

Similar results are obtained for the HT1080 and M4T1 malignant cells (**Figures 1** **and** **2**).

In the non-conditioned medium, cellular invasion is maximal (42% with HT1080 cells and 49% with M4T1 cells). In the medium conditioned by dermal fibroblasts, cellular invasion is of 42% for HT1080 cells and 53% for M4T1 cells. In themedium conditioned by gingival fibroblasts, cellular invasion is of 22% for HT1080 cells and 15% for M4T1 cells.

Thus, a medium conditioned by gingival fibroblasts inhibits cellular invasion of basal lamina extracts in comparison with a medium conditioned with dermal fibroblasts or with a non-conditioned medium.

### 2. Conditioned medium from human gingival fibroblasts inhibit cellular invasion by malignant cells in collacien I

Similar results are obtained for the HT1080 and M4T1 malignant cells (**Figures 3** **and** **4**).

In the non-conditioned medium, cellular invasion is maximal (38% with HT1080 cells and 48% with M4T1 cells). In the medium conditioned by dermal fibroblasts, cellular invasion is of 42% for HT1080 cells and 53% for M4T1 cells. In the medium conditioned by gingival fibroblasts, cellular invasion is of 21% for HT1080 cells and 15% for M4T1 cells.

Thus, a medium conditioned by gingival fibroblasts inhibits cellular invasion of collagen I in comparison with a medium conditioned with dermal fibroblasts or with a non-conditioned medium.

## Claims

1. A gingival fibroblast-derived product for use for preventing or treating melastatic cancer in an individual, wherein cancer cell invasion is inhibited and wherein said gingival fibroblast-derived product is selected from the group consisting of gingival fibroblast whole cells, a gingival fibroblast culture, a gingival fibroblast extract and a gingival fibroblast conditioned medium.

2. The gingival fibroblast-derived product for the use according to claim 1, for preventing or treating tumor invasion.

3. The gingival fibroblast-derived product for the use according to claim 1 or 2, wherein the gingival fibroblast-derived product is administered by local injection.

4. The gingival fibroblast-derived product for the use according to claim 1 or 2, wherein the gingival fibroblast-derived product is administered by a route selected from the group consisting of the oral route, the subcutaneous route, the intravenous route, and the intramuscular route.

5. The gingival fibroblast-derived product for the use according to any of claims 1 to 4, wherein the gingival fibroblast-derived product is a gingival fibroblast conditioned medium.

6. The gingival fibroblast-derived product for the use according to any of claims 1 to 5, wherein the gingival fibroblast-derived product is obtained from gingival fibroblasts taken from the individual.

7. The gingival fibroblast-derived product for the use according to any of claims 1 to 6, wherein said cancer is a metastatic cancer or a cancer liable to form metastasis.

8. The gingival fibroblast-derived product for the use according to any of claims 1 to 7, wherein said cancer is a solid tumor.

9. The gingival fibroblast-derived product for the use according to any of claims 1 to 8, wherein metastasis of said cancer is prevented or treated.

10. The gingival fibroblast-derived product for the use according to any of claims 1 to 9, wherein said cancer is cancer of connective tissues.

## Patentansprüche

1. Aus Zahnfleisch-Fibroblasten gewonnenes Produkt zum Gebrauch in der Vorbeugung und Behandlung von Krebsmetastasen in einem Patienten, wobei die Invasion von Krebszellen verhütet wird und wobei das genannte aus Zahnfleisch-Fibroblasten gewonnene Produkt aus der Gruppe gewählt ist, bestehend aus ganzen Zahnfleisch-Fibroblasten-Zellen, einer Zahnfleisch-Fibroblasten-Kultur, einem Zahnfleisch-Fibroblasten-Auszug und einem mit Zahnfleisch-Fibroblasten behandelten Medium.

2. Zahnfleisch-Fibroblasten-Produkt zum Gebrauch nach Patentanspruch 1 zur Verhütung oder Behandlung von Tumorinvasion.

3. Zahnfleisch-Fibroblasten-Produkt zum Gebrauch nach Patentanspruch 1 oder 2, wobei das Zahnfleisch-Fibroblasten-Produkt durch lokale Injektion verabreicht wird.

4. Zahnfleisch-Fibroblasten-Produkt zum Gebrauch nach Patentanspruch 1 oder 2, wobei das Zahnfleisch-Fibroblasten-Produkt in einer Weise verabreicht wird, ausgewählt aus der Gruppe, bestehend aus oraler Verabreichung, subkutaner Verabreichung, intravenöser Verabreichung und intramuskulärer Verabreichung.

5. Zahnfleisch-Fibroblasten-Produkt zum Gebrauch nach irgendeinem der Patentansprüche 1 bis 4, wobei das Zahnfleisch-Fibroblasten-Produkt ein mit Zahnfleisch-Fibroblasten behandeltes Medium ist.

6. Zahnfleisch-Fibroblasten-Produkt zum Gebrauch nach irgendeinem der Patentansprüche 1 bis 5, wobei das Zahnfleisch-Fibroblasten-Produkt aus Zahnfleisch-Fibroblasten gewonnen wurde, die dem Patienten entnommen wurden.

7. Zahnfleisch-Fibroblasten-Produkt zum Gebrauch nach irgendeinem der Patentansprüche 1 bis 6, wobei die genannte Krebsform eine Metastasen bildende Krebsform ist oder eine zur Metastasenbildung neigende Krebsform.

8. Zahnfleisch-Fibroblasten-Produkt zum Gebrauch nach irgendeinem der Patentansprüche 1 bis 7, wobei der genannte Krebs ein fester Tumor ist.

9. Zahnfleisch-Fibroblasten-Produkt zum Gebrauch nach irgendeinem der Patentansprüche 1 bis 8, wobei Metastasenbildung des genannten Krebses verhütet oder behandelt wird.

10. Zahnfleisch-Fibroblasten-Produkt zum Gebrauch nach irgendeinem der Patentansprüche 1 bis 9, wobei der genannte Krebs ein Bindegewebskrebs ist.

## Revendications

1. Produit dérivé de fibroblastes gingivaux pour son utilisation dans la prévention ou le traitement des cancers métastasiques chez un individu, dans laquelle l'invasion des cellules cancéreuses est inhibée et dans laquelle ledit produit dérivé de fibroblastes gingivaux est sélectionné dans le groupe constitué de cellules entières de fibroblastes gingivaux, d'une culture de fibroblastes gingivaux, d'un extrait de fibroblastes gingivaux et d'un milieu conditionné de fibroblastes gingivaux.

2. Produit dérivé de fibroblastes gingivaux pour son utilisation selon la revendication 1, pour prévenir ou traiter une invasion tumorale.

3. Produit dérivé de fibroblastes gingivaux pour son utilisation selon la revendication 1 ou 2, dans laquelle le produit dérivé de fibroblastes gingivaux est administré par injection locale.

4. Produit dérivé de fibroblastes gingivaux pour son utilisation selon la revendication 1 ou 2, dans laquelle le produit dérivé de fibroblastes gingivaux est administré par une voie sélectionnée dans le groupe constitué de la voie orale, de la voie sous-cutanée, de la voie intraveineuse, et de la voie intramusculaire.

5. Produit dérivé de fibroblastes gingivaux pour son utilisation selon l'une des revendications 1 à 4, dans laquelle le produit dérivé de fibroblastes gingivaux est un milieu conditionné de fibroblastes gingivaux.

6. Produit dérivé de fibroblastes gingivaux pour son utilisation selon l'une des revendications 1 à 5, dans laquelle le produit dérivé de fibroblastes gingivaux est obtenu à partir de fibroblastes gingivaux prélevés chez l'individu.

7. Produit dérivé de fibroblastes gingivaux pour son utilisation selon l'une des revendications 1 à 6, dans laquelle ledit cancer est un cancer métastasique ou un cancer susceptible de former des tumeurs.

8. Produit dérivé de fibroblastes gingivaux pour son utilisation selon l'une des revendications 1 à 7, dans laquelle ledit cancer est une tumeur solide.

9. Produit dérivé de fibroblastes gingivaux pour son utilisation selon l'une des revendications 1 à 8, dans laquelle on traite ou on prévient la métastase dudit cancer.

10. Produit dérivé de fibroblastes gingivaux pour son utilisation selon l'une des revendications 1 à 9, dans laquelle ledit cancer est un cancer des tissus connectifs.
